# EUROPEAN PATENT SPECIFICATION

(11) **EP 3 856 708 B1**
(45) Date of publication and mention of the grant of the patent: **25.10.2023**
(21) Application number: 19864878.4
(22) Date of filing: 19.09.2019
(51) Int. Cl.: C07C 29/38, B01J 23/02, C07C 31/24, C07C 29/78, C07C 41/40, C07C 43/13, C07C 41/09

(54) **PROCESS FOR PRODUCTION OF PENTAERYTHRITOL WITH AN INCREASED YIELD OF DI-PENTAERYTHRITOL**
VERFAHREN ZUR HERSTELLUNG VON PENTAERYTHRIT MIT ERHÖHTER DIPENTAERYTHRITAUSBEUTE
PROCÉDÉ DE PRODUCTION DE PENTAÉRYTHRITOL AVEC UN RENDEMENT ACCRU DE DI-PENTAÉRYTHRITOL

(30) Priority: 28.09.2018 SE 1830268
(43) Date of publication of application: 04.08.2021
(73) Proprietor: Perstorp AB, 284 80 Perstorp (SE)
(72) Inventor: MÅNSSON, Christoffer, 286 92 Örkelljunga (SE); FINNHULT, Daniel, 265 32 Åstorp (SE)
(74) Representative: Hoffmann Eitle
(86) International application number: PCT/SE2019/050883
(87) International publication number: WO 2020/067963

(56) References cited:
- GB-A- 958 654
- US-A- 2 186 272
- US-A- 5 741 956
- US-A1- 2012 178 973

## Description

### FIELD OF THE INVENTION

The present invention refers to a process for production of pentaerythritol with an increased yield of di-pentaerythritol, wherein acetaldehyde, formaldehyde, calcium hydroxide and a formose inhibitor are reacted in an aqueous solution.

### BACKGROUND OF THE INVENTION

Di-Pentaerythritol is an important raw material for many environmentally friendly applications. It is used in high-solids decorative alkyds in order to meet legislation demands on maximum content of VOC in paints. Its high functionality (six primary hydroxyl groups) improves the drying and hardness development. Di-Pentaerythritol improves properties also in radiation curing monomers, synthetic lubricants and flame retardants.

As there is today no industrially useful method for direct synthesis of di-pentaerythritol, it is obtained as a by-product in the manufacture of pentaerythritol. Because of the difference in solubility between the two compounds, they can be separated by fractional crystallization.

Pentaerythritol is prepared by reacting formaldehyde and acetaldehyde in the presence of a base.

The most commonly used bases are sodium hydroxide and calcium hydroxide; which will result in sodium formate and calcium formate respectively as by-products. Calcium formate has a considerably higher market value than sodium formate and it can be used for example as a food or feed additive or as an additive in cement.

GB958654A discloses a continuous process to prepare pentaerythritol and dipentaerythritol using calcium hydroxide or sodium hydroxide in the absence of formose inhibitor, without separate addition of formaldehyde or pausing during the reactants addition.

US5741956A discloses a batch process to prepare pentaerythritol and dipentaerythritol using sodium hydroxide.

The calcium hydroxide based reaction occurs according to *Figure 1* below:

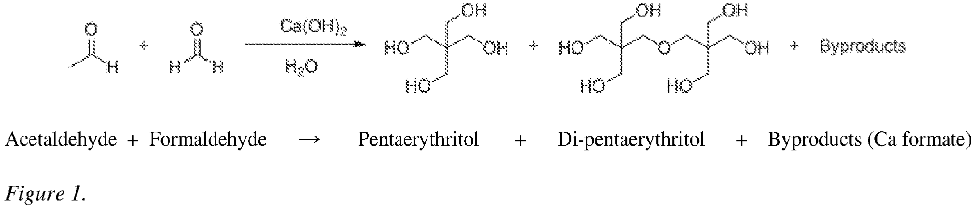

This process is typically run with a molar ratio of formaldehyde : acetaldehyde of about 5.9 : 1 and with all the formaldehyde added at the start of the reaction. At these conditions, the synthetic yield of di-pentaerythritol is about 3%, calculated against acetaldehyde. To improve the di-pentraerythritol yield, the molar ratio formaldehyde : acetaldehyde can be reduced or the dosing of the formaldehyde can be changed, so that formaldehyde is dosed continuously to the reaction. Both of these actions will result in decreased equivalents of formaldehyde being available during the reaction. This can give di-pentaerythritol synthetic yields of more than 4%, calculated against acetaldehyde. However, under these conditions, a side-reaction called formose will emerge. In the formose reaction, formaldehyde is consumed in an autocatalytic cycle forming higher sugars. These higher sugars will decompose to form new molecules of glycolaldehyde. The reaction happens in the presence of a divalent cation, such as calcium, by stabilizing enediol intermediates. It starts very slowly, but once the reaction is running the formed intermediates will catalyze the reaction. The formose reaction is illustrated in *Figure 2* below.

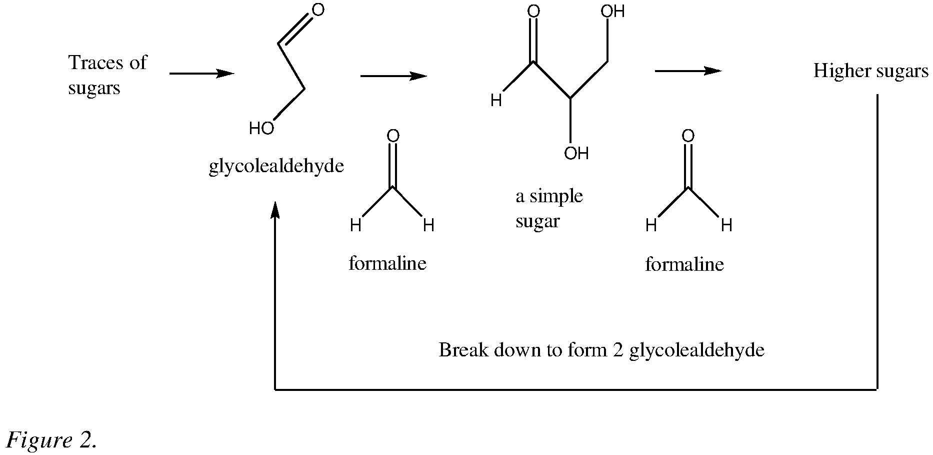

Measuring the glycolaldehyde concentration is a good indicator of whether there is formose or not. Severe formose will also be visible as it will turn the synthesis solution yellow and then brown.

The occurrence of formose will decrease the yield, increase the amount of by-products and give a product with overall lower quality. Thus, there is a need for a calcium-based process for production of pentaerythritol wherein the yield of di-pentaerythritol is increased while at the same time the formose reaction is avoided.

### DETAILED DESCRIPTION OF THE INVENTION

As described above, the industrial route to produce di-pentaerythritol today is as a by-product in the production of pentaerythritol, by reacting acetaldehyde and formaldehyde in alkaline medium. This reaction is very well known and well described in the literature. In the normal process for producing pentaerythritol, the molar ratio of formaldehyde to acetaldehyde is increased well beyond the stoichiometric ratio of 4:1. This is in order to avoid formation of higher homologues of pentaerythritol.

If instead it is desired to maximize the yield of di-pentaerythritol, the molar ratio of formaldehyde to acetaldehyde should be decreased towards the stoichiometric. This can be achieved by simply decreasing the molar ratio formaldehyde : acetaldehyde and adding all the formaldehyde to the reaction solution at the beginning of the reaction. Another way to decrease the molar equivalents of formaldehyde being present during the reaction is to add a part of the formaldehyde continuously to the reaction solution, instead of adding all the formaldehyde at the start. When decreased amounts of formaldehyde are used, acrolein is formed as an intermediate. Acrolein is reactive against pentaerythritol and the other intermediates and di-pentaerythritol is formed.

However, in the case where calcium hydroxide is used as the base in the reaction, decreasing the formaldehyde equivalents will after some time cause problems with the so-called formose reaction appearing. In the formose reaction, formaldehyde is consumed in an autocatalytic cycle forming higher sugars. Once this cycle has been initiated the formose reaction will be reinforced and the formaldehyde will be consumed in that reaction, at the expense of the desired reaction where pentaerythritol and di-pentaerythritol are formed.

Despite the problems appearing when trying to raise the yield of di-pentaerythritol in the calcium-based process for production of pentaerythritol, there is a strong demand to be able to use calcium hydroxide instead of sodium hydroxide as the base in the process. This is because calcium formate, that is formed as a by-product in the calcium-based process, has a much higher value on the market than sodium formate, that is formed in the sodium-based process. Calcium formate is used for example as a food or feed additive or as an additive in cement.

The present invention has revealed a calcium-based process for production of pentaerythritol with very high yields of di-pentaerythritol, which can be run without problems with the formose reaction emerging.

The present invention refers to a process for production of pentaerythritol with an increased yield of di-pentaerythritol, said process using calcium hydroxide as catalyst, the process comprising the following steps:
a) Adding water, less than half of the total amount of formaldehyde and a formose inhibitor to a reactor and heating the reaction solution to 25-40 °C
b) Continuously adding acetaldehyde, calcium hydroxide and at least half of the total amount of formaldehyde to the reactor during a time period of 20-80 minutes and at the same time increasing the temperature to 45-55 °C
c) Pausing the continuous dosing of formaldehyde after 5-20 minutes of dosing, the pause in dosing being 5-20 minutes long
d) Keeping the temperature at 45-55 °C during 10-30 minutes for postreaction and then neutralizing the solution
e) Adding a flocculating agent to remove impurities from the calcium hydroxide and adjusting the pH to below 4.5
f) Purifying the pentaerythritol and di-pentaerythritol respectively with conventional methods, including a formaldehyde stripping and recirculation step and a final fractional crystallization step
wherein the molar ratio formaldehyde : acetaldehyde in said process is < 6 : 1 and the molar ratio calcium hydroxide : acetaldehyde in said process is < 0.65 : 1 and wherein the synthetic yield of di-pentaerythritol in said process is at least 4%, calculated against acetaldehyde.

The synthetic yield of di-pentaerythritol in the process according to the present invention is preferably at least 7%, calculated against acetaldehyde. Di-pentaerythritol yields as high as 8%, calculated against acetaldehyde, have been achieved with the process according to the invention. The formose inhibitor used in the process according to the present invention is preferably selected from the group consisting of tungstic acid, boric anhydride, boric acid, dysprosium oxide, samarium oxide, manganese II acetyl acetonate, manganese III acetate, sodium metaborate tetra-hydrate and manganese II acetate tetra-hydrate.

According to a preferred embodiment of the present invention, said formose inhibitor is tungstic acid. The concentration of said tungstic acid is preferably 50-200 ppm by weight, based on the total reaction solution.

The process according to the present invention starts by adding water, formaldehyde and the formose inhibitor to a reactor. Typically, about 1/3 of the formaldehyde is added at the start of the reaction. The temperature of the reaction solution is then increased to about 25-40 °C. When the desired temperature is reached, the continuous dosing of acetaldehyde, calcium hydroxide and the rest of the formaldehyde is started. Acetaldehyde, calcium hydroxide and at least half of the amount of formaldehyde are dosed continuously over a time period of 20-80 minutes, preferably 40-60 minutes.

It was surprisingly found that pausing the continuous dosing of formaldehyde for some time increases the yield of di-pentaerythritol in the process. Experiments show that the timing and length of the pause in the formaldehyde dosing is of great importance for the final result. The excess of formaldehyde needs to be low enough at the right time during the reaction, in order to favor an increased formation of di-pentaerythritol. The continuous dosing of formaldehyde should be paused after 5-20 minutes of dosing, for a time period of about 5-20 minutes.

We have seen in experiments leading to the present invention that simply decreasing the stoichiometric excess of formaldehyde (without adjusting the dosing profile) can lead to problems during the work up process, the product turning mushy.

During the continuous dosing of acetaldehyde, calcium hydroxide and formaldehyde, the temperature is ramped from about 25-40 °C to about 45-55 °C. After the dosing, the temperature is kept at 45-55 °C for 10-30 minutes for postreaction, where after the reaction solution is neutralized to about pH 7, preferably with formic acid. After the neutralizing step a flocculating agent is added to remove impurities from the calcium hydroxide. The pH is thereafter preferably set to below 4.5 with for example formic acid.

According to one embodiment of the present invention the residuals of the formose inhibitor, remaining in the synthesis solution after the flocculation step are removed from the reaction solution by means of ion exchange. When tungstic acid is used as formose inhibitor, the residual tungsten is preferably removed with an anionic ion exchanger. Pentaerythritol and di-pentaerythritol are then stepwise purified by conventional methods, including a formaldehyde stripping and recirculation step and a final fractional crystallization step.

During the work leading to the present invention, it has been noted that different grades of calcium hydroxide are more or less suitable to use in the process according to the invention. It is believed that factors like particle size and degree of crystallinity are important. Experiments have shown that pre-treating the calcium hydroxide with sonication might further reduce the risk for formose occurring.

The present invention is illustrated in the below Embodiment Examples, which are to be construed as merely illustrative and not limiting in any way:
*- Example 1 (comparative) illustrates synthesis of pentaerythritol according to the "normal" method, i.e. not with an improved yield of di-pentaerythritol*
- *Example 2 (comparative) illustrates synthesis of pentaerythritol with an increased yield of di-pentaerythritol (formaldehyde is dosed over time but no formose inhibitor is used)*
- *Example 3 (comparative) illustrates synthesis of pentaerythritol with an increased yield of di-pentaerythritol (formaldehyde is dosed over time and formose inhibitor is used)*
*- Example 4 (according to the invention) illustrates synthesis of pentaerythritol with an increased yield of di-pentaerythritol (formaldehyde dosing is paused and formose inhibitor is used)*

### EMBODIMENT EXAMPLES

### Example 1 (comparative): Synthesis of pentaerythritol - normal method

Recipe for the synthesis:

| **Compound** | **Amount (g)** | **Mole** | **Molar ratio against acetaldehyde** |
|---|---|---|---|
| Acetaldehyde (99.6%) | 51,1 | 1,13 | 1:1 |
| Formaldehyde (50%) | 401,5 | 6,69 | 1:5,9 |
| Ca(OH)₂ (33%) | 154,1 | 0,67 | 1:0,59 |

The synthesis was carried out in an automated Syrris Atlas lab system. The lime (calcium hydroxide) that was used was supplied from Schaefer Kalk and had a purity of -96%. Formaldehyde and water was added to the reactor and the solution was heated to 30°C. When the desired temperature was reached the dosing of acetaldehyde and calcium hydroxide was started. Acetaldehyde was pumped in with a flow of ~1.3 ml/min for 50 min. Calcium hydroxide was pumped in with a flow of 3.1 g/ml for 50 minutes. During the dosing the temperature was ramped from 30°C to 48°C for 50 minutes. After the dosing the temperature was kept at 48°C for 20 minutes for postreaction. After the postreaction the solution was neutralized to ~pH 7 with formic acid. A flocculating agent was then added to remove impurities from the calcium hydroxide. The pH of the solution was adjusted to pH < 4.5 with formic acid.

### Example 2 (comparative): Synthesis of pentaerythritol - formaldehyde is dosed over time

Recipe for the synthesis:

| **Compound** | **Amount (g)** | **Mole** | **Molar ratio against acetaldehyde** |
|---|---|---|---|
| Acetaldehyde (99.6%) | 74,4 | 1,7 | 1:1 |
| Formaldehyde (19,5%) | 1540,8 | 10,0 | 1:5,9 |
| Ca(OH)₂ (33%) | 243,4 | 1,05 | 1:0,62 |
| Water | 77,7 | | |

The synthesis was carried out in an automated Syrris Atlas lab system. The lime (calcium hydroxide) that was used was supplied from Schaefer and had a purity of -96%. Approximately ~1/3 of the formaldehyde and water was added to the reactor. The solution was heated to 30°C. When the desired temperature was reached the dosing of acetaldehyde, calcium hydroxide and the rest of the formaldehyde was started. Acetaldehyde was pumped in with a flow of ~1.9 ml/min, calcium hydroxide was pumped in with a flow of ~4.9 ml/min and formaldehyde was pumped in with a flow of ~19.9 ml/min. Pumping of all three substances was continued for 50 minutes. During the dosing, the temperature was ramped from 30°C to 48°C over 50 minutes. After the dosing the temperature was kept at 48°C for 20 minutes for postreaction. After the postreaction the solution was neutralized to ~pH 7 with formic acid. A flocculating agent was then added to remove impurities from the calcium hydroxide. The pH of the solution was adjusted to pH < 4.5 with formic acid.

### Example 3 (comparative): Synthesis of pentaerythritol - formaldehyde is dosed over time, formose inhibitor is used

Recipe for the synthesis:

| **Compound** | **Amount (g)** | **Mole** | **Molar ratio against acetaldehyde** |
|---|---|---|---|
| Acetaldehyde (99.6%) | 74,4 | 1,7 | 1:1 |
| Formaldehyde (19,5%) | 1540,8 | 10,0 | 1:5,9 |
| Ca(OH)₂ (33%) | 243,4 | 1,05 | 1:0,62 |
| Water | 77,7 | | |

The synthesis was done according to the process described in Example 2, except that 100 ppm tungstic acid (by weight, based on the total reaction solution) was added together with the ~1/3 of the formaldehyde and water.

### Example 4 (according to the invention): Synthesis of pentaerythritol - formaldehyde is dosed over time with a pause and formose inhibitor is used

Recipe for the synthesis:

| **Compound** | **Amount (g)** | **Mole** | **Molar ratio against acetaldehyde** |
|---|---|---|---|
| Acetaldehyde (99.6%) | 74,4 | 1,7 | 1:1 |
| Formaldehyde (19,5%) | 1540,8 | 10,0 | 1:5,9 |
| Ca(OH)₂ (33%) | 243,4 | 1,05 | 1:0,62 |
| Water | 77,7 | | |

The synthesis was carried out in an automated Syrris Atlas lab system. The lime (calcium hydroxide) that was used was supplied from Schaefer and had a purity of -96%. Approximately ~1/3 of the formaldehyde, water and 100 ppm tungstic acid (by weight, based on the total reaction solution) was added to the reactor. The solution was heated to 30°C. When the desired temperature was reached the dosing of acetaldehyde, calcium hydroxide and the rest of the formaldehyde was started. Acetaldehyde was pumped in with a flow of ~1.9 ml/min, calcium hydroxide was pumped in with a flow of ~4.9 ml/min and formaldehyde was pumped in with a flow of ~19.9 ml/min. After 10 minutes of pumping, the pumping of formaldehyde was paused and continued after 10 minutes. Pumping of all three substances was continued for in total 50 minutes, so the pumping of formaldehyde stopped 10 minutes after the pumping of acetaldehyde and calcium hydroxide. During the dosing the temperature was ramped from 30°C to 48°C over 50 minutes. After the dosing was completed the temperature was kept at 48°C for 20 minutes for postreaction. After the postreaction the solution was neutralized to ~pH 7 with formic acid. A flocculating agent was then added to remove impurities from the calcium hydroxide. The pH of the solution was adjusted to pH < 4.5 with formic acid.

### Results

The average synthetic yield of Di-pentaerythritol (% against acetaldehyde) and the average content of glycolaldehyde (ppm by weight, based on the total synthesis solution) in the synthesis solution were determined with HPLC/MS. The values reported in *Table 1* are average values from in total 31 individual synthesis experiments.

**Table 1.**

| **Example** | **Average yield Di-Penta (% against acetaldehyde)** | **Average Glycol aldehyde content (ppm by weight, based on total solution)** | **Color (after reaction)** |
|---|---|---|---|
| 1 | 3.3 | 0 | No |
| 2 | 3.9 | 274 | Light yellow - brown in most syntheses |
| 3 | 4.1 | 1.6 | No |
| 4 | 7.7 | 2 | No |

As can be seen in *Table 1*, the average synthetic yield of di-pentaerythritol in *Example 1* is 3.3%, which is in line with values reported in the literature for the "normal" pentaerythritol process. In *Example 2*, formaldehyde is dosed over time, thus keeping down the molar equivalents of formaldehyde during the reaction, which results in an average synthetic yield of di-pentaerythritol of 3.9 %. However, in this reaction the formose is evident, with a high average value of glycolaldehyde and the reaction solution turning yellow or even brown. In *Exampe 3,* where a formose inhibitor is used, the average synthetic yield of di-pentaerythritol is on the same level as in *Example 2*, but there are no signs of formose occurring.

In *Example 4,* where formaldehyde is dosed continuously and where the dosing is paused for 10 minutes after 10 minutes reaction, the average synthetic yield of di-pentaerythritol is as high as 7.7 % (calculated against acetaldehyde), which is a very high yield in a calcium-based pentaerythritol synthesis. At the same time, there are no signs of formose taking place in the reaction solution.

## Claims

1. A process for production of pentaerythritol with an increased yield of di-pentaerythritol, said process using calcium hydroxide as catalyst, the process comprising the following steps:
a) Adding water, less than half of the total amount of formaldehyde and a formose inhibitor to a reactor and heating the reaction solution to 25-40 °C
b) Continuously adding acetaldehyde, calcium hydroxide and at least half of the amount of formaldehyde to the reactor during a time period of 20-80 minutes and at the same time increasing the temperature to 45-55 °C
c) Pausing the continuous dosing of formaldehyde after 5-20 minutes of dosing, the pause in dosing being 5-20 minutes long
d) Keeping the temperature at 45-55 °C during 10-30 minutes for postreaction and then neutralizing the solution
e) Adding a flocculating agent to remove impurities from the calcium hydroxide and adjusting the pH to below 4.5
f) Purifying the pentaerythritol and di-pentaerythritol respectively with conventional methods, including a formaldehyde stripping and recirculation step and a final fractional crystallization step
wherein the molar ratio formaldehyde : acetaldehyde in said process is < 6 : 1 and the molar ratio calcium hydroxide : acetaldehyde in said process is < 0.65 : 1 and wherein the synthetic yield of di-pentaerythritol in said process is at least 4%, calculated against acetaldehyde.

2. A process according to claim 1 **characterized in, that** the synthetic yield of di-pentaerythritol in said process is at least 7%, calculated against acetaldehyde.

3. A process according to claim 1 or 2 **characterized in, that** said formose inhibitor is selected from the group consisting of tungstic acid, boric anhydride, boric acid, dysprosium oxide, samarium oxide, manganese II acetyl acetonate, manganese III acetate, sodium metaborate tetra-hydrate and manganese II acetate tetra-hydrate.

4. A process according to claim 3 **characterized in, that** said formose inhibitor is tungstic acid.

5. A process according to claim 4 **characterized in, that** the concentration of said tungstic acid is 50-200 ppm by weight, based on the total reaction solution.

6. A process according to any of the claims 1-5 **characterized in, that** acetaldehyde, calcium hydroxide and at least half of the amount of formaldehyde are dosed continuously over a time period of 40-60 minutes.

7. A process according to any of the claims 1-6 **characterized in, that** residuals of said formose inhibitor, remaining after the reaction, are removed from the reaction solution by means of ion exchange.

8. A process according to claim 7 **characterized in, that** said formose inhibitor is tungstic acid and that the residual tungsten is removed with an anionic ion exchanger.

9. A process according to any of the claims 1-8 **characterized in, that** the calcium hydroxide is pre-treated with sonication.

## Patentansprüche

1. Verfahren zur Herstellung von Pentaerythrit mit erhöhter Dipentaerythritausbeute, wobei das Verfahren Calciumhydroxid als Katalysator verwendet, das Verfahren umfassend die folgenden Schritte:
a) die Zugabe von Wasser, weniger als der Hälfte der Gesamtmenge an Formaldehyd und einen Formoseinhibitor zu einem Reaktor und das Erwärmen der Reaktionslösung auf 25-40 °C
b) die kontinuierliche Zugabe von Acetaldehyd, Calciumhydroxid und mindestens der Hälfte der Menge an Formaldehyd zu dem Reaktor über einen Zeitraum von 20-80 Minuten und das gleichzeitige Erhöhen der Temperatur auf 45-55 °C
c) das Unterbrechen der kontinuierlichen Dosierung von Formaldehyd nach 5-20 Minuten Dosierung, wobei die Dosierungspause 5-20 Minuten lang ist
d) das Halten der Temperatur bei 45-55 °C für 10-30 Minuten für die Nachreaktion und das anschließende Neutralisieren der Lösung
e) die Zugabe eines Flockungsmittels zur Entfernung von Verunreinigungen aus dem Calciumhydroxid und das Einstellen des pH-Werts auf unter 4,5
f) das Aufreinigen des Pentaerythrits bzw. Dipentaerythrits mit herkömmlichen Methoden, einschließlich eines Formaldehyd-Stripping- und Rückführungsschritts und eines abschließenden fraktionierten Kristallisationsschritts,
worin das Molverhältnis Formaldehyd : Acetaldehyd in dem Verfahren < 6 : 1 beträgt und das Molverhältnis Calciumhydroxid : Acetaldehyd in dem Verfahren < 0,65 : 1 beträgt und worin die Syntheseausbeute von Dipentaerythrit in dem Verfahren mindestens 4%, gegen Acetaldehyd berechnet, beträgt.

2. Verfahren gemäß Anspruch 1, **dadurch gekennzeichnet, dass** die Syntheseausbeute von Dipentaerythrit in dem Verfahren mindestens 7%, gegen Acetaldehyd berechnet, beträgt.

3. Verfahren gemäß Anspruch 1 oder 2, **dadurch gekennzeichnet, dass** der Formoseinhibitor aus der Gruppe ausgewählt ist, bestehend aus Wolframsäure, Borsäureanhydrid, Borsäure, Dysprosiumoxid, Samariumoxid, Mangan-II-Acetylacetonat, Mangan-III-Acetat, Natriummetaborat-Tetrahydrat und Mangan-II-Acetat-Tetrahydrat.

4. Verfahren gemäß Anspruch 3, **dadurch gekennzeichnet, dass** der Formoseinhibitor Wolframsäure ist.

5. Verfahren gemäß Anspruch 4, **dadurch gekennzeichnet, dass** die Konzentration der Wolframsäure 50-200 ppm nach Gewicht, bezogen auf die gesamte Reaktionslösung, beträgt.

6. Verfahren gemäß einem der Ansprüche 1-5, **dadurch gekennzeichnet, dass** Acetaldehyd, Calciumhydroxid und mindestens die Hälfte der Menge an Formaldehyd kontinuierlich über einen Zeitraum von 40-60 Minuten dosiert werden.

7. Verfahren gemäß einem der Ansprüche 1-6, **dadurch gekennzeichnet, dass** nach der Reaktion verbleibende Reste des Formoseinhibitors mittels Ionenaustausch aus der Reaktionslösung entfernt werden.

8. Verfahren gemäß Anspruch 7, **dadurch gekennzeichnet, dass** der Formoseinhibitor Wolframsäure ist und verbleibendes Wolfram mit einem anionischen Ionentauscher entfernt werden.

9. Verfahren gemäß einem der Ansprüche 1-8, **dadurch gekennzeichnet, dass** das Calciumhydroxid mit Ultraschall vorbehandelt wird.

## Revendications

1. Procédé de production de pentaérythritol avec un rendement accru en dipentaérythritol, ledit procédé utilisant de l'hydroxyde de calcium comme catalyseur, le procédé comprenant les étapes suivantes :
a) l'ajout d'eau, moins que la moitié de la quantité totale de formaldéhyde et un inhibiteur de formose dans un réacteur et le chauffage de la solution réactionnelle à 25-40°C
b) l'ajout en continu d'acétaldéhyde, d'hydroxyde de calcium et d'au moins la moitié de la quantité de formaldéhyde dans le réacteur pendant une période de temps de 20-80 minutes et, en même temps, l'augmentation de la température à 45-55°C
c) la mise en pause de l'introduction continue de formaldéhyde après 5-20 minutes d'introduction, la mise en pause de l'introduction étant de 5-20 minutes
d) le maintien de la température à 45-55°C pendant 10-30 minutes à des fins de post-réaction, et ensuite la neutralisation de la solution
e) l'ajout d'un agent de floculation pour éliminer les impuretés de l'hydroxyde de calcium et l'ajustement du pH en dessous de 4,5
f) la purification du pentaérythritol et du dipentaérythritol respectivement avec des procédés classiques, comportant une étape d'extraction et de remise en circulation du formaldéhyde et une étape finale de cristallisation fractionnée
dans lequel le rapport molaire formaldéhyde : acétaldéhyde dans ledit procédé est < 6 : 1 et le rapport molaire hydroxyde de calcium : acétaldéhyde dans ledit procédé est < 0,65 : 1 et dans lequel le rendement de synthèse du dipentaérythritol dans ledit procédé est au moins de 4%, calculé par rapport à l'acétaldéhyde.

2. Procédé selon la revendication 1, **caractérisé en ce que** le rendement de synthèse du dipentaérythritol dans ledit procédé est au moins de 7%, calculé par rapport à l'acétaldéhyde.

3. Procédé selon la revendication 1 ou la revendication 2, **caractérisé en ce que** ledit inhibiteur de formose est sélectionné dans le groupe consistant en l'acide tungstique, l'anhydride borique, l'acide borique, l'oxyde de dysprosium, l'oxyde de samarium, l'acétylacétonate de manganèse II, l'acétate de manganèse III, le métaborate de sodium tétrahydraté et l'acétate de manganèse II tétrahydraté.

4. Procédé selon la revendication 3, **caractérisé en ce que** ledit inhibiteur de formose est l'acide tungstique.

5. Procédé selon la revendication 4, **caractérisé en ce que** la concentration dudit acide tungstique est de 50-200 ppm en poids, sur la base de la solution réactionnelle totale.

6. Procédé selon l'une quelconque des revendications 1 à 5, **caractérisé en ce que** l'acétaldéhyde, l'hydroxyde de calcium et au moins la moitié de la quantité de formaldéhyde sont introduits en continu sur une période de temps de 40-60 minutes.

7. Procédé selon l'une quelconque des revendications 1 à 6, **caractérisé en ce que** les résidus dudit inhibiteur de formose, restant après la réaction, sont éliminés de la solution réactionnelle au moyen d'un échange d'ions.

8. Procédé selon la revendication 7, **caractérisé en ce que** ledit inhibiteur de formose est l'acide tungstique et **en ce que** le tungstène résiduel est éliminé avec un échangeur d'ions anioniques.

9. Procédé selon l'une quelconque des revendications 1 à 8, **caractérisé en ce que** l'hydroxyde de calcium est prétraité par sonification.
